# EUROPEAN PATENT APPLICATION

(11) **EP 3 184 019 A1**
(43) Date of publication of application: **28.06.2017**
(21) Application number: 15833536.4
(22) Date of filing: 03.07.2015
(51) Int. Cl.: A61B 1/00

(54) **TRANSMISSION MECHANISM, RAISING DEVICE, AND INSERTION EQUIPMENT**

(30) Priority: 19.08.2014 JP 2014166841
(71) Applicant: Olympus Corporation, Tokyo 192-8507 (JP)
(72) Inventor: YAMAYA, Koji, Hachioji-shi Tokyo 192-8507 (JP)
(74) Representative: Schicker, Silvia
(86) International application number: PCT/JP2015/069313
(87) International publication number: WO 2016/027574

(57) **Abstract**

A transmission mechanism includes a pulling and pressing member including a distal end portion coupled with a raising stand and a proximal end portion coupled with a raising operation unit and a containing portion containing the pulling and pressing member with the distal end portion of the pulling and pressing member projecting therefrom. The transmission mechanism further includes an elastic member that the distal end portion side of the pulling and pressing member projecting from the containing portion is inserted into the cylindrical elastic member.

## Description

### Technical Field

The present invention relates to a transmission mechanism that transmits an operating force of a raising operation unit to a raising stand to raise up the raising stand that raises an insertion object to be inserted into an inserting apparatus, a raising device including the transmission mechanism, and the insertion apparatus including the raising device.

### Background Art

Examples of ordinary medical endoscopes are gastroscopes and duodenum scopes. For example, a treatment instrument and a guide member that guides the treatment instrument to the affected part function as insertion objects to be inserted into an inserting unit of the endoscope. The insertion object collects or treats the affected part, in the state where the inserting unit is inserted into the body cavity of the patient, the affected part is observed with the endoscope, and the insertion object is inserted into the insertion unit. The insertion object is raised by a raising device disposed in the endoscope, and is opposed to the affected part.

The raising device includes a raising stand that rises up to raise the insertion object to be inserted into the inserting unit from a distal end opening portion disposed at a distal end of the inserting unit, and a raising operation unit that operates the raising stand by remote control to raise the raising stand. The raising operation unit is disposed in an operating unit that is coupled with the inserting unit and operates the inserting unit. The raising device further includes a transmission mechanism that is coupled with the raising stand and the raising operation unit and transmits an operating force of the raising operation unit to the raising stand. The raising operation unit pulls or presses a raising operation wire disposed on the transmission mechanism, to adjust the raising angle of the raising stand and adjust the direction of the insertion object.

When the inserting unit is inserted into the body cavity, the inserting unit contacts contaminated liquid such as body fluid. For this reason, the contaminated liquid adheres to the raising stand, the raising operation wire, and the coupling member coupled to the raising stand and the distal end portion of the raising operation wire. The used endoscope must be washed to prevent transmission of infectious diseases. Because above members of the raising device described have fine, a washing work to remove the contaminated liquid adhered to the members requires much labor. In particular, because the raising operation wire is thin, the washing work to remove the contaminated liquid adhered to the raising operation wire requires much labor, and has low washing efficiency.

For this reason, for example, in Patent Literature 1, to prevent adhesion of contaminated liquid, a raising operation wire is contained inside the distal end portion of the insertion unit, to prevent exposure of the raising operation wire.

### Citation List

### Patent Literature

Patent Literature 1: Jpn. Pat. Appln. KOKAI Publication No. 8-056900

### Summary of Invention

### Technical Problem

In Patent Literature 1, the wire containing unit in which the raising operation wire is contained is covered with a cover or the like. However, in consideration of washing work, the wire containing unit requires watertightness to be secured with an O-ring or the like. This structure tends to increase the diameter of the distal end portion of the inserting unit.

The present invention has been made in view of these circumstances, and the object of the present invention is to provide a transmission mechanism, a raising device, and an inserting apparatus that prevent adhesion of contaminated liquid, and improve the washing efficiency, while suppressing increase a diameter of the distal end portion of the inserting unit.

### Solution to Problem

A transmission mechanism according to an aspect of the present invention is coupled to a raising stand and a raising operation unit operating the raising stand, and transmits an operating force of the raising operation unit to the raising stand, the raising stand raises up a tool inserted through an inserting unit inserted into a lumen from an opening portion disposed in the inserting unit by rotation, and the transmission mechanism comprises: a pulling and pressing member including a distal end portion coupled with the raising stand and a proximal end portion coupled with the raising operation unit, and pulling or pressing the raising stand by an operation of the raising operation unit to rotate the raising stand; a containing portion containing the pulling and pressing member with the distal end portion of the pulling and pressing member projecting therefrom; and a cylindrical elastic member including a distal end portion connected in a watertight state to the raising stand, and a proximal end portion connected in a watertight state to the containing portion, and expanding and contracting, the distal end portion side of the pulling and pressing member projecting from the containing portion is inserted into the cylindrical elastic member.

### Advantageous Effects of Invention

The present invention provides a transmission mechanism, a raising device, and an inserting apparatus that prevent adhesion of contaminated liquid, and improve the washing efficiency, while suppressing an increase in the diameter of the inserting unit.

### Brief Description of Drawings

FIG. 1 is a schematic diagram of an insertion system according to an embodiment of the present invention;
FIG. 2A is a top view of a distal end hard portion according to an embodiment;
FIG. 2B is a front view of the distal end hard portion as viewed from an arrow 2B side illustrated in FIG. 2A;
FIG. 3A is a diagram illustrating a transmission mechanism in a maximum laid state;
FIG. 3B is a diagram illustrating the transmission mechanism in a maximum raised state;
FIG. 4A is a cross-sectional view taken along line 4A-4A illustrated in FIG. 3A, illustrating coupling between a pulling and pressing member and a raising stand;
FIG. 4B is a diagram illustrating Modification 1 of coupling between the pulling and pressing member and the raising stand;
FIG. 4C is a diagram illustrating Modification 2 of coupling between the pulling and pressing member and the raising stand;
FIG. 4D is a diagram illustrating Modification 3 of coupling between the pulling and pressing member and the raising stand;
FIG. 5A is a diagram illustrating a length of an elastic region in a natural length;
FIG. 5B is a diagram illustrating a length of the elastic region in the maximum laid state;
FIG. 5C is a diagram illustrating a length of the elastic region in the maximum raised state;
FIG. 6A is a diagram illustrating a modification of the length of the elastic member, and illustrating the transmission mechanism in a laid state;
FIG. 6B is a diagram illustrating a modification of the length of the elastic member, and illustrating the transmission mechanism in a raised state;
FIG. 7A is a diagram illustrating a length of the elastic region in a natural length;
FIG. 7B is a diagram illustrating a length of the elastic region in the maximum laid state;
FIG. 7C is a diagram illustrating a length of the elastic region in the maximum raised state;
FIG. 8A is a diagram illustrating Modification 1 of the elastic member;
FIG. 8B is a diagram illustrating Modification 2 of the elastic member;
FIG. 8C is a diagram illustrating Modification 3 of the elastic member;
FIG. 8D is a diagram illustrating a state where the elastic member of any one of Modifications 1 to 3 illustrated in FIG. 8A, FIG. 8B and FIG. 8C contracts;
FIG. 9A is a diagram illustrating Modification 1 of the elastic member fixed in a watertight state; and
FIG. 9B is a diagram illustrating Modification 2 of the elastic member fixed in a watertight state.

### Description of Embodiments

Embodiments of the present invention will be explained hereinafter with reference to drawings.

### [First Embodiment]

### [Configuration]

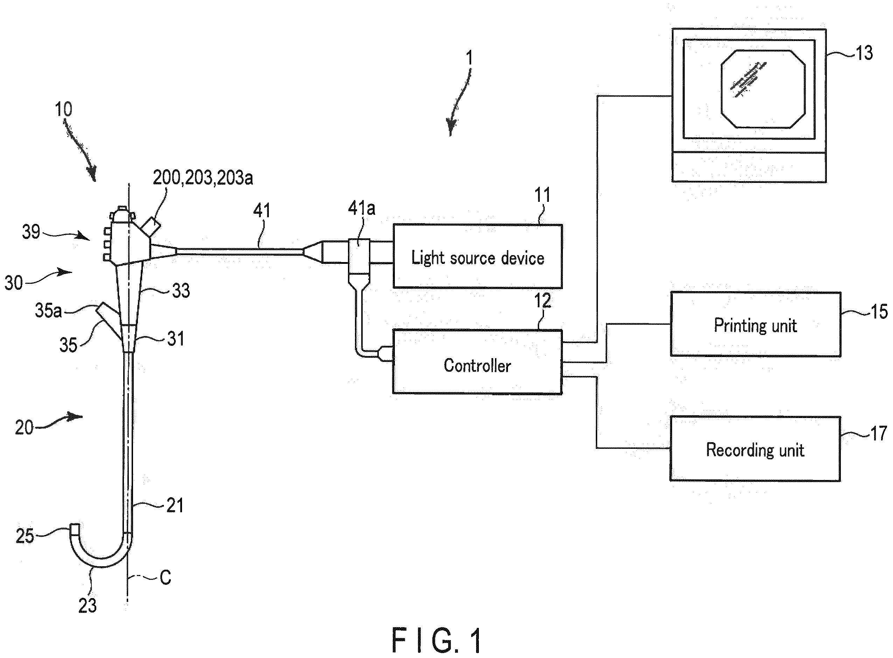

An embodiment will be explained hereinafter with reference to FIG. 1, FIG. 2A, FIG. 2B, FIG. 3A, FIG. 3B, FIG. 4A, FIG. 4B, FIG. 4C, FIG. 4D, FIG. 5A, FIG. 5B, and FIG. 5C. In some drawings, illustration of some members is omitted to clarify the illustration.

### [Insertion System 1]

As illustrated in FIG. 1, an insertion system 1 includes an inserting apparatus 10, a light source device 11 that supplies illumination light to the inserting apparatus 10, and a controller 12 that includes a signal converter (not illustrated) that converts an electrical signal transmitted from an imaging unit (not illustrated) disposed in the inserting apparatus 10 into an image signal. The insertion system 1 further includes a display unit 13 that displays the image signal, a printing unit 15 that prints an image displayed on the display unit 13, and a recording unit 17 that records the image signal.

### [Inserting Apparatus 10]

The inserting apparatus 10 illustrated in FIG. 1 functions as, for example, an endoscope inserted into a lumen in the body cavity. The inserting apparatus 10 according to the present embodiment is, for example, a side-viewing endoscope that inserts a treatment instrument into a duodenal papilla or biliary pancreas. The inserting apparatus 10 according to the present embodiment may be a direct-view endoscope, or an endoscope that inserts a treatment instrument into a region other than the regions described above.

The inserting apparatus 10 according to the present embodiment is explained as a medical endoscope, for example, but is not necessarily limited to such. In addition to a medical endoscope, the inserting apparatus 10 may suitably be an industrial endoscope, or an insertion tool such as a catheter and an overtube, which includes no illumination optical system or observation optical system.

An insertion object referred to hereinafter is a tool including a treatment instrument (not illustrated) inserted into an inserting unit 20 of the inserting apparatus 10 described above, and a guide member (not illustrated) that guides the treatment instrument to the affected part.

As illustrated in FIG. 1, the inserting apparatus 10 has a longitudinal axis C. The inserting apparatus 10 includes the inserting unit 20 that extended along the longitudinal axis C, and an operating unit 30 that is disposed in a proximal end portion of the inserting unit 20 and operates the inserting unit 20.

As illustrated in FIG. 1, the inserting unit 20 includes a flexible tube portion 21 that is coupled with the operating unit 30 and has flexibility, a bent portion 23 that is coupled with a distal end of the flexible tube portion 21 and is bendable with respect to the longitudinal axis C, and a distal end hard portion 25 that is coupled with a distal end of the bent portion 23.

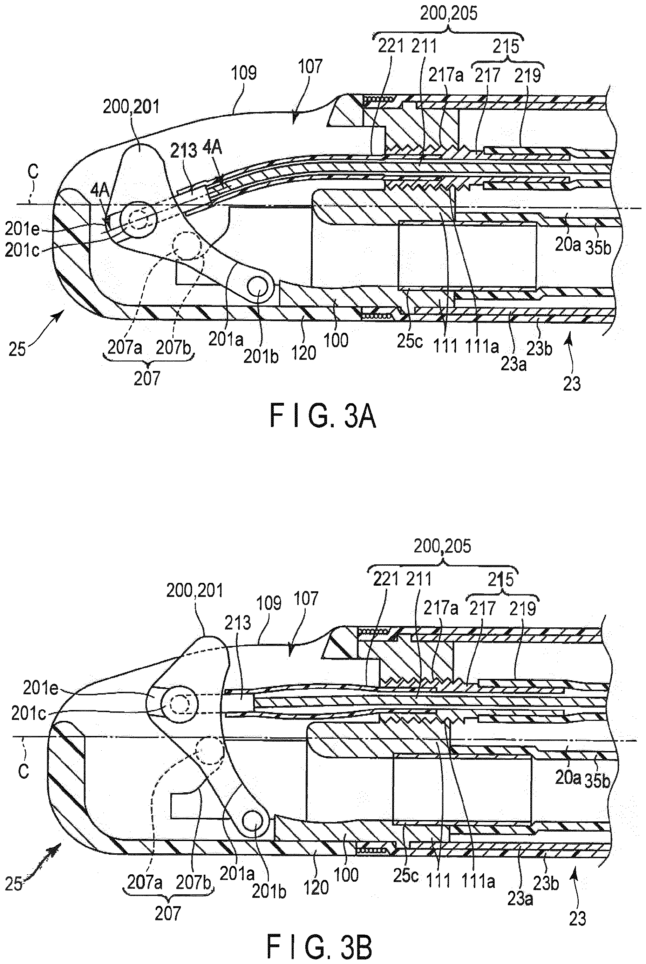

As illustrated in FIG. 3A and FIG. 3B, the distal end hard portion 25 is coupled with the bent portion 23, with a joint ring 23a of the bent portion 23 fitted into a proximal end portion of a main body portion 100 of the distal end hard portion 25. The joint ring 23 is covered with a cylindrical cover member 23b, and a distal end portion of the cover member 23b is fixed in a watertight state onto the main body portion 100 with threads and by adhesion. The configuration of the distal end hard portion 25 will be described later.

As illustrated in FIG. 1, the operating unit 30 includes a main body portion 31 from which the flexible tube portion 21 extends, a grasping portion 33 that is grasped by the operator who operates the inserting apparatus 10, and a universal cord 41 connected with the grasping portion 33.

As illustrated in FIG. 1, the grasping portion 33 includes an insertion-object inserting portion 35, a bending operation portion (not illustrated) to perform an operation to bend the bent portion 23, and a switch portion 39 to operate the observation unit and the illumination unit.

As illustrated in FIG. 1, the insertion-object inserting portion 35 is disposed at an end portion of the grasping portion 33, and includes an inserting port 35a to insert the insertion object into the inserting unit 20 of the inserting apparatus 10.

The inserting port 35a is connected with a proximal end portion of a channel 35b illustrated in FIG. 3A and FIG. 3B. The channel 35b is disposed inside the inserting unit 20, and disposed from the flexible tube portion 21 to the distal end hard portion 25 through the bent portion 23.

A distal end portion of the channel 35b is fixed in a water-tight state to a cylindrical connecting member 25c of the main body portion 100, in a state where the distal end portion of the channel 35b is opposed to a raising stand 201 disposed inside the distal end hard portion 25 and the channel 35b communicates with a communicating portion 107.

The channel 35b is disposed apart from a contained member space portion 20a which is disposed in inside the inserting unit 20 in a watertight state from the outside, and in which a contained member is disposed. The contained member includes a cable of the observation unit and a light-guide tube of the illumination unit.

The universal cord 41 further includes a connecting portion 41a that is directly or indirectly connected to the light source device 11 and the controller 12. The connecting portion 41a is disposed at an end portion of the universal cord 41.

### [Distal End Hard Portion 25]

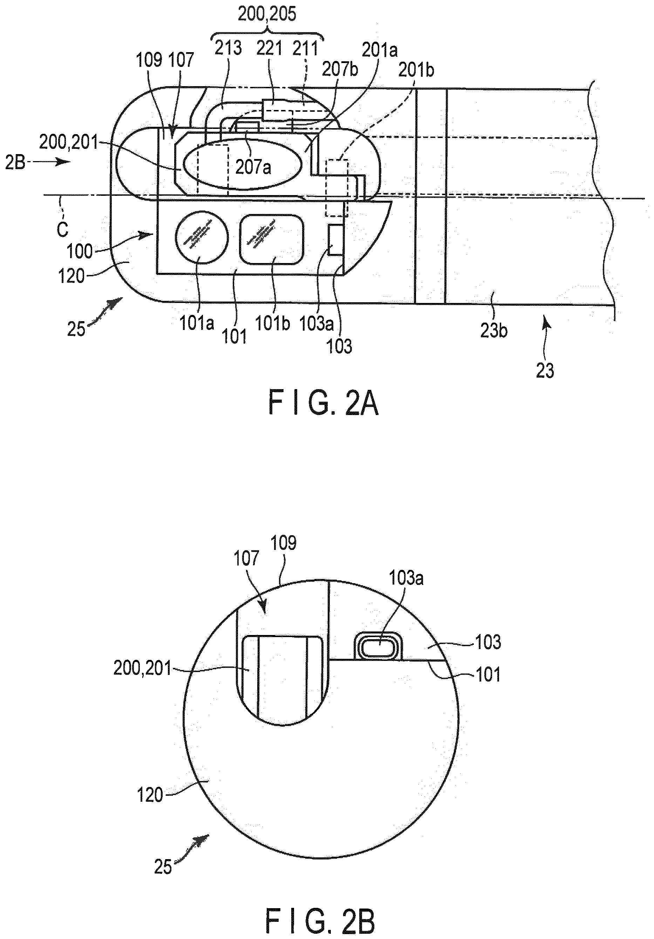

As illustrated in FIG. 2A, FIG. 2B, FIG. 3A, and FIG. 3B, the distal end hard portion 25 includes, for example, the main body portion 100 formed of metal such as SUS, and a cover portion 120 that covers the main body portion 100 and has an electrical insulation property.

As illustrated in FIG. 2A and FIG. 2B, the distal end hard portion 25 includes a plane-shaped observation surface portion 101 disposed in part of the main body portion 100 and on the side of the main body portion 100, and exposed to the outside, and a wall portion 103 that is part of the cover portion 120.

The observation surface portion 101 is disposed along the longitudinal axis C, and the wall portion 103 is orthogonal to the longitudinal axis C (observation surface portion 101).

The main body portion 100 includes an illumination window portion 101a of the illumination unit and an observation window portion 101b of the observation unit that are disposed in the observation surface portion 101, and a nozzle portion 103a that is disposed in a state of projecting from the wall portion 103.

The observation window portion 101b and the illumination window portion 101a are mutually arranged on the same plane, and the nozzle portion 103a is disposed with an opening end opposed to the observation window portion 101b and the illumination window portion 101a. In the direction of the longitudinal axis C, the observation window portion 101b is disposed between the illumination window portion 101a and the nozzle portion 103a.

The illumination window portion 101a illuminates the illumination light supplied from the light source device 11 to the affected part, and the observation window portion 101b observes the affected part. The nozzle portion 103a ejects fluid toward the illumination window portion 101a and the observation window portion 101b, to wash and remove the contaminated fluid adhered to the illumination window portion 101a and the observation window portion 101b.

As illustrated in FIG. 2A, FIG. 2B, FIG. 3A, and FIG. 3B, the distal end hard portion 25 further includes the communicating portion 107 that contains the raising stand 201, and a distal end opening portion 109 formed on the side surface of the distal end hard portion 25.

As illustrated in FIG. 3A and FIG. 3B, the communicating portion 107 communicates with the outside through the distal end opening portion 109. With such configuration, the communicating portion 107 contains the raising stand 201, in a state where the raising stand 201 is exposed to the outside.

As illustrated in FIG. 3A and FIG. 3B, the main body portion 100 includes a wall portion 111 as part of the main body portion 100, the wall portion 111 separates the contained member space portion 20a from the communicating portion 107 in the longitudinal axis C direction, and is disposed inside the inserting unit 20.

As described above, the communicating portion 107 is separated from the contained member space portion 20a by the wall portion 111, to secure watertightness of the contained member space portion 20a.

### [Raising Device 200]

As illustrated in FIG. 1, FIG. 2A, FIG. 2B, FIG. 3A, and FIG. 3B, the inserting apparatus 10 further includes a raising device 200 that raises the insertion object to be inserted into the inserting unit 20 from the distal end opening portion 109 disposed at the distal end of the inserting unit 20.

As illustrated in FIG. 1, FIG. 2A, FIG. 2B, FIG. 3A, and FIG. 3B, the raising device 200 includes the raising stand 201 that raises up the insertion object inserted through the inserting unit 20 inserted into the tube cavity from the distal end opening portion 109 at the distal end of the inserting unit 20 by rotation, and a raising operation unit 203 that is disposed in the operating unit 30 coupled with the inserting unit 20 and operates the raising stand 201 by remote control to raise or lay down the raising stand 201 by rotation.

The raising device 200 further includes a transmission mechanism 205 that is coupled to the raising stand 201 and the raising operation unit 203, and transmits an operating force of the raising operation unit 203 to the raising stand 201 as the force for rotating the raising stand 201. The raising operation unit 203 and the transmission mechanism 205 function as the raising operation mechanism that operates the raising stand 201.

### [Raising Stand 201]

As illustrated in FIG. 2A, FIG. 2B, FIG. 3A, and FIG. 3B, the raising stand 201 is attached to the main body portion 100, in a state of being rotatable with respect to the main body portion 100.

The raising stand 201 is rotated between a maximum laid state (see FIG. 3A) in which the raising stand 201 is contained in the communicating portion 107 in a laid state, and a maximum raised state (see FIG. 3B) in which the raising stand 201 is raised.

The raising stand 201 controls the leading direction of the insertion object inserted through the channel 35b, according to the raising state thereof.

As illustrated in FIG. 2A, FIG. 3A, and FIG. 3B, the raising stand 201 includes a guide surface 201a having a curved surface shape and guiding the insertion object, and a raising shaft 201b that functions as a rotation shaft in raising. The guide surface 201a is disposed on a surface of the raising stand 201.

The raising shaft 201b extends through the base (proximal end portion) of the raising stand 201, and is supported by the main body portion 100. The raising shaft 201b is covered with a flexible resin or an O-ring (not illustrated). This configuration prevents infiltration of contaminated fluid into the base and the main body portion 100. The raising stand 201 is rotated at a desired angle around the raising shaft 201b serving as the center, and thereby the raising stand 201 is displaced in a direction in which the guide surface 201a is raised up. With displacement of the raising stand 201, the insertion object guided by the guide surface 201a is raised.

As illustrated in FIG. 3A and FIG. 3B, the raising device 200 further includes a regulating unit 207 that regulates a rotation amount of the raising stand 201.

The regulating unit 207 includes a regulating member 207a that is disposed on a side surface of the raising stand 201 and moved together with rotation of the raising stand 201, and a regulating portion 207b that serves as part of the main body portion 100 and against which the regulating member 207a abuts.

The regulating member 207a is a bar-shaped member that is disposed, for example, in parallel with the raising shaft 201b, and rotatable around the axis of the regulating member 207a. The regulating member 207a abuts against one end portion (proximal end portion) of the regulating portion 207b when the raising stand 201 is raised, and abuts against the other end portion (distal end portion) of the regulating portion 207b when the raising stand 201 is laid down.

As illustrated in FIG. 3A, the regulating member 207a abuts against the distal end portion of the regulating portion 207b, and thereby the raising stand 201 is stopped in the maximum laid state. As illustrated in FIG. 3B, the regulating member 207a abuts against the proximal end portion of the regulating portion 207b, and thereby the raising stand 201 is stopped in the maximum raised state as illustrated in FIG. 3B. With such configuration, the regulating unit 207 defines the maximum raised state, the maximum laid state, and the raising range of the raising stand 201.

### [Raising Operation Unit 203]

The raising operation unit 203 illustrated in FIG. 1 pulls the raising stand 201 via the transmission mechanism 205 to switch the raising stand 201 to the raised state, and presses the raising stand 201 via the transmission mechanism 205 to switch the raising stand 201 to the laid state.

An operating lever 203a of the raising operation unit 203 is rotated by a user operation. The rotating operation of the operating lever 203a is transmitted to a coupling member (not illustrated) of the raising operation unit 203, and thereby the coupling member is moved forward or backward. The forward or backward force is transmitted to the raising stand 201 via the transmission mechanism 205, and rotates the raising stand 201.

### [Transmission Mechanism 205]

As illustrated in FIG. 2A, FIG. 3A, and FIG. 3B, the transmission mechanism 205 is disposed inside the operating unit 30 and inside the inserting unit 20.

The transmission mechanism 205 includes a pulling and pressing member 211 that includes a distal end portion coupled with the raising stand 201 and a proximal end portion coupled with the raising operation unit 203, and a containing portion 215 that contains the pulling and pressing member 211 in a state where the distal end portion of the pulling and pressing member 211 projects from the containing portion 215 to the outside. The containing portion 215 contains most of the pulling and pressing member 211 including the proximal end portion of the pulling and pressing member 211 and excluding the distal end portion of the pulling and pressing member 211.

The transmission mechanism 205 further includes an elastic member 221 including a distal end portion that is connected in a watertight state to the raising stand 201 and a proximal end portion that is connected in a watertight state to the containing portion 215.

### [Pulling and Pressing Member 211 and Coupling Member 213]

As illustrated in FIG. 3A and FIG. 3B, the pulling and pressing member 211 is interposed between a coupling member (not illustrated) of the raising operation unit 203 and the raising stand 201 in the direction of the longitudinal axis C.

The pulling and pressing member 211 is disposed along the longitudinal axis C. The distal end portion of the pulling and pressing member 211 pierces the wall portion 111, and is coupled with the raising stand 201 with the coupling member 213 described later. The pulling and pressing member 211 other than the distal end portion thereof is disposed from the raising stand 201 and inside the bent portion 23, the flexible tube portion 21, and the operating unit 30 and contained in the contained member space portion 20a, and not disposed in the communicating portion 107.

As illustrated in FIG. 3A and FIG. 3B, the pulling and pressing member 211 is moved forward and backward along the longitudinal axis C serving as the axis direction of the transmission mechanism 205, by operation of the raising operation unit 203 and, by the forward or backward movement, pulls the raising stand 201 to raise up the raising stand 201, or presses the raising stand 201 to lay down the raising stand 201. The pulling and pressing member 211 pulls or presses the raising stand 201, to rotate the raising stand 201.

As illustrated in FIG. 3A and FIG. 3B, when the pulling and pressing member 211 presses the raising stand 201 toward the distal end of the distal end hard portion 25, the raising stand 201 is contained (laid down). When the pulling and pressing member 211 pulls the raising stand 201, the raising stand 201 is raised up. The pulling and pressing member 211 has a desired length.

The pulling and pressing member 211 includes a line-shaped member such as a wire member. The pulling and pressing member 211 is formed of, for example, metal. The pulling and pressing member 211 is bendable.

As illustrated in FIG. 2A, FIG. 3A, and FIG. 3B, the distal end portion of the pulling and pressing member 211 is indirectly coupled with the raising stand 201, via coupling member 213 that is coupled with the raising stand 201 and the distal end portion of the pulling and pressing member 211. The coupling member 213 is included in the transmission member 205.

As illustrated in FIG. 3A and FIG. 3B, the coupling member 213 is disposed in the communicating portion 107.

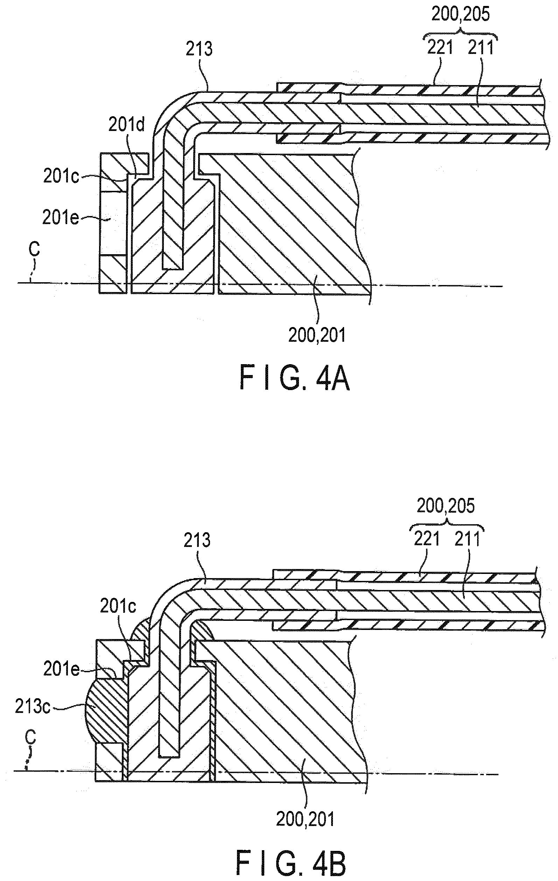

As illustrated in FIG. 3A, FIG. 3B, and FIG. 4A, the coupling member 213 has a cylindrical member, and the distal end portion of the pulling and pressing member 211 is inserted into the coupling member 213.

In the coupling member 213 into which the distal end portion of the pulling and pressing member 211 is inserted, the coupling member 213 is fixed to the distal end portion of the pulling and pressing member 211 with solder (not illustrated), for example. With such configuration, the distal end portion of the pulling and pressing member 211 is coupled in a watertight state with the coupling member 213.

As illustrated in FIG. 4A, in the coupling member 213 into which the distal end portion of the pulling and pressing member 211 is inserted, the coupling member 213 is bendable in a direction of being shifted from the axial direction of the pulling and pressing member 211. Specifically, the coupling member 213 is bendable by, for example, 90° from the longitudinal axis C serving as the axial direction of the pulling and pressing member 211 toward the orthogonal direction, together with the distal end portion of the pulling and pressing member 211. After the distal end portion of the coupling member 213 is inserted into an inserting port portion 201c that is disposed along the side surface and the orthogonal direction of the raising stand 201, the proximal end portion of the coupling member 213 is bent in the longitudinal axis C direction from the orthogonal direction.

In this manner, according to the present embodiment, the distal end portion of the pulling and pressing member 211 is coupled with the raising stand 201 via the coupling member 213, and the distal end portion of the pulling and pressing member 211 is covered with the coupling member 213 and is not exposed to the outside.

As illustrated in FIG. 4A, the inserting port portion 201c is slightly larger than the distal end portion of the coupling member 213, and a space 201d is formed between the inserting port portion 201c and the distal end portion of the coupling member 213.

With such configuration, when the raising stand 201 is rotated at the distal end portion of the coupling member 213 inserted into the inserting port portion 201c, and the distal end portion of the coupling member 213 is relatively shifted with respect to the inserting port portion 201c around the axis of the distal end portion. Specifically, the distal end portion of the coupling member 213 is not fixed in the inserting port portion 201c, but is capable of sliding the inserting port portion 201c around the axis of the distal end portion of the coupling member 213.

Specifically, when the raising stand 201 is rotated, the distal end portion of the coupling member 213 is not rotated around the axis. With rotation of the raising stand 201, the position of the inserting port portion 201c in the direction of around the axial is shifted with respect to the distal end portion of the coupling member 213. With the configuration described above, the raising stand 201 is enabled to raise by, for example, 90° with respect to the longitudinal axis C direction.

As illustrated in FIG. 4A, the raising stand 201 is provided with a coupling part washing port portion 201e, to enable easy washing of the distal end portion of the coupling member 213 and the inserting port portion 201c.

According to the operative method, it suffices that the raising stand 201 rises by up to substantially 45° at the maximum with respect to the longitudinal axis C direction. In this case, Modifications 1, 2, and 3 as follows are established in coupling of the pulling and pressing member 211 and the raising stand 201.

As Modification 1 illustrated in FIG. 4B, in the distal end portion of the coupling member 213 inserted into the inserting port portion 201c, the distal end portion of the coupling member 213 is fixed in the inserting port portion 201c with an adhesive 213c, for example. The adhesive 213c is filled into the inserting port portion 201c and the coupling part washing port portion 201e.

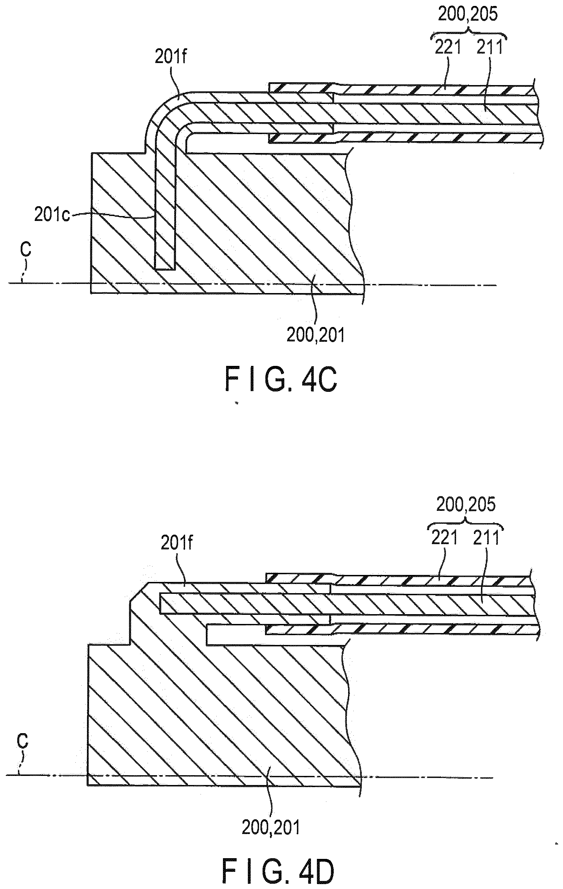

As Modification 2 illustrated in FIG. 4C, the distal end portion of the pulling and pressing member 211 is bent in a direction being shifted from the axial direction of the pulling and pressing member 211, and directly coupled with the raising stand 201. Specifically, the distal end portion of the pulling and pressing member 211 is bent from the axial direction of the pulling and pressing member 211 toward the orthogonal direction that is orthogonal to the axial direction, and directly coupled with the raising stand 201. With such configuration, the raising stand 201 includes an attachment portion 201f that is bent by 90°, for example, from the longitudinal axis C direction toward the orthogonal direction to attach the pulling and pressing portion 211 to the raising stand 201. The attachment portion 201f is united with the raising stand 201.

The attachment portion 201f has a cylindrical shape, and communicates with the inserting port portion 201c. The distal end portion of the pulling and pressing member 211 is inserted into the attachment portion 201f and the inserting port portion 201c, and thereby bent by 90°, for example, from the longitudinal axis C direction toward the orthogonal direction.

The distal end portion of the pulling and pressing member 211 is fixed to the raising stand 201 including the attachment portion 201f and the inserting port portion 201c by soldering or the like. With such configuration, the distal end portion of the pulling and pressing member 211 is directly coupled in a watertight state with the raising stand 201.

As described above, in the present modification, the pulling and pressing member 211 is directly coupled with the raising stand 201, and directly fixed in a watertight state to the raising stand 201.

As shown by Modification 3 illustrated in FIG. 4D, the distal end portion of the pulling and pressing member 211 is directly coupled with the raising stand 201, in a state of being disposed along the axial direction of the pulling and pressing member 211. With such configuration, the attachment member 201f is disposed on the side surface of the raising stand 201, and disposed along the longitudinal axis C direction. The inserting port portion 201c is omitted. Modification 3 is substantially the same as Modification 2 except for the points described above.

As described above, the distal end portion of the pulling and pressing member 211 is indirectly or directly coupled with the raising stand 201 via the coupling member 213, in a state where no part of the pulling and pressing member 211 is exposed to the outside.

### [Containing portion 215]

As illustrated in FIG. 3A and FIG. 3B, the containing portion 215 is built in the inserting unit 20, and disposed the wall portion 111 and inside the contained member space portion 20a. With this configuration, the containing portion 215 is disposed coaxially with the contained member space portion 20a and the communicating portion 107 in the longitudinal axis C direction.

As illustrated in FIG. 3A and FIG. 3B, the containing portion 215 as described above includes a cylindrical mouthpiece portion 217, and a cylindrical member 219 that is connected in a watertight state with a proximal end portion of the mouthpiece portion 217 in a state of communicating with the mouthpiece portion 217.

When the pulling and pressing member 211 is inserted into the mouthpiece portion 217 and the cylindrical member 219, the mouthpiece portion 217 and the cylindrical member 219 cover the pulling and pressing member 211. In the state where the pulling and pressing member 211 is inserted into the mouthpiece portion 217 and the cylindrical member 219, a clearance portion is secured between the pulling and pressing member 211 and the mouthpiece portion 217 and the cylindrical member 219. The mouthpiece portion 217 and the cylindrical member 219 function as an insertion channel portion through which the pulling and pressing member 211 is inserted, and are disposed along the longitudinal axis C direction.

The mouthpiece portion 217 functions as a guide hole portion to guide the pulling and pressing member 211 to be coupled with the raising stand 201 to the contained member space portion 20a.

As illustrated in FIG. 3A and FIG. 3B, the mouthpiece portion 217 includes a distal end portion including a fixed portion 217a that is fixed in a watertight state in a wall hole portion 111a of the wall portion 111, and a proximal end portion disposed in the contained member space portion 20a.

The wall hole portion 111a extends through the wall portion 111 in the longitudinal axis C direction. The fixed portion 217a is screwed in a watertight state into the wall portion 111. The distal end portion of the mouthpiece portion 217 is not located in the communicating portion 107, but disposed inside the wall hole portion 111a. When the cylindrical mouthpiece portion 217 is connected with the cylindrical member 219, the proximal end portion of the mouthpiece portion 217 is inserted into the distal end portion of the cylindrical member 219, and the mouthpiece portion 217 communicates with the cylindrical member 219.

An external circumferential surface of the proximal end portion of the mouthpiece portion 217 is in close contact with an internal circumferential surface of the distal end portion of the cylindrical member 219. Because the proximal end portion is disposed in the contained member space portion 20a, the external circumferential surface of the proximal end portion of the mouthpiece portion 217 may be watertight or not watertight with the internal circumferential surface of the distal end portion of the cylindrical member 219. The mouthpiece portion 217 is formed of, for example, metal.

As illustrated in FIG. 3A and FIG. 3B, the cylindrical member 219 is disposed between the coupling member (not illustrated) of the raising operation unit 203 and the mouthpiece portion 217 in the longitudinal axis C direction, and in the contained member space portion 20a.

When the pulling and pressing member 211 is inserted into the cylindrical member 219, the cylindrical member 219 covers the pulling and pressing member 211 in the contained member space portion 20a. The cylindrical member 219 prevents the pulling and pressing member 211 from contacting the contained member and wearing down in the contained member space portion 20a.

The cylindrical member 219 functions as a guide member to guide the pulling and pressing member 211 from the mouthpiece portion 217 to the raising operation unit 203. The cylindrical member 219 is formed of, for example, resin.

### [Elastic Member 221]

As illustrated in FIG. 3A and FIG. 3B, the elastic member 221 includes a cylindrical (tube-shaped) member into which the distal end portion of the pulling and pressing member 211 projecting from the mouthpiece portion 217 of the containing portion 215 is inserted.

In the state where the pulling and pressing member 211 is inserted into the elastic member 221 and the elastic member 221 covers the pulling and pressing member 211, a clearance portion is secured between the pulling and pressing member 211 and the elastic member 221. The elastic member 221 prevents the pulling and pressing member 211 from contacting the inserted member and wearing down in the communicating portion 107.

The elastic member 221 functions as an insertion channel portion through which the pulling and pressing member 211 is inserted in the state of being covered with the elastic member 221 and not being exposed to the outside. The elastic member 221 has a smooth internal circumferential surface and a smooth external circumferential surface.

As illustrated in FIG. 3A, FIG. 3B, FIG. 4A, and FIG. 4B, when the distal end portion of the pulling and pressing member 211 is coupled with the coupling member 213, the distal end portion of the elastic member 221 is connected in a watertight state to the coupling member 213.

Specifically, in a state where the coupling member 213 communicates with the elastic member 221, the proximal end portion of the coupling member 213 is inserted into the distal end portion of the elastic member 221. The external circumferential surface of the proximal end portion is adhered to the internal circumferential surface of the distal end portion of the elastic member 221.

As described above, the elastic member 221 is indirectly coupled with the raising stand 201 via the coupling member 213, and the pulling and pressing member 211 is covered with the coupling member 213 and the elastic member 221, and not exposed to the outside.

As illustrated in FIG. 4C and FIG. 4D, when the distal end portion of the pulling and pressing member 211 is directly coupled with the raising stand 201, the distal end portion of the elastic member 221 is connected in a watertight state to the attachment portion 201f of the raising stand 201.

Specifically, in a state where the attachment portion 201f communicates with the elastic member 221, the proximal end portion of the attachment portion 201f is inserted into the distal end portion of the elastic member 221. The external circumferential surface of the proximal end portion is adhered to the internal circumferential surface of the distal end portion. As described above, the elastic member 221 is directly coupled with the raising stand 201, and directly fixed in a watertight state to the raising stand 201.

As illustrated in FIG. 3A and FIG. 3B, in a state where the elastic member 221 communicates with the mouthpiece portion 217 (guide hole portion), the proximal end portion of the elastic member 221 is inserted into the mouthpiece portion 217. The external circumferential surface of the proximal end portion is adhered and fixed to the internal circumferential surface of the mouthpiece portion 217.

As described above, the proximal end portion of the elastic member 221 is inserted into the distal end portion of the mouthpiece portion 217, and fixed in a watertight state to the distal end portion of the mouthpiece portion 217.

The elastic member 221 may be exchangeable, as long as watertightness is secured.

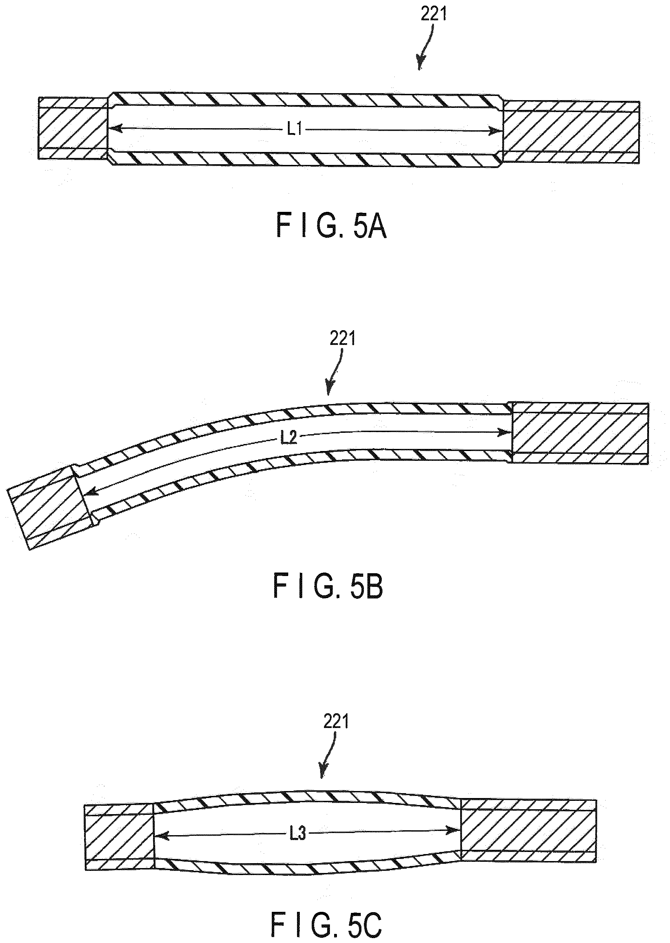

As illustrated in FIG. 3A, FIG. 3B, FIG. 5A, FIG. 5B, and FIG. 5C, the elastic member 221 expands and contracts in the axial direction of the elastic member 221, by pulling and pressing of the pulling and pressing member 211.

In the present embodiment, in the laid state, because the pressing force of the pulling and pressing member 211 acts on the elastic member 221 via the raising stand 201 and the coupling member 213, the elastic member 221 expands to be longer than its natural length. In the raised state, because the pulling force of the pulling and pressing member 211 acts on the elastic member 221 via the raising stand 201 and the coupling member 213, the elastic member 221 contracts to be shorter than its natural length.

With such structure, the elastic member 221 includes an elastic region that expands and contracts in a region excluding the distal end portion and the proximal end portion. In this case, the following length relation applies.

Suppose that L1 is a length of the elastic region in a state before the elastic member 221 is mounted to the main body portion 100, that is, in its natural length, as illustrated in FIG. 5A.

Suppose that L2 is a length of the elastic region in the maximum laid state, in a state where the elastic region is mounted to the main body portion 100, that is, the proximal end portion is fixed to the mouthpiece portion 217 and the distal end portion is fixed to the coupling member 213, as illustrated in FIG. 3A and FIG. 5B. Illustration of L2 is omitted in FIG. 3A, to clarify the illustration.

Suppose that L3 is a length of the elastic region in the maximum raised state, in a state where the elastic region is mounted to the main body portion 100, that is, the proximal end portion is fixed to the mouthpiece portion 217 and the distal end portion is fixed to the coupling member 213, as illustrated in FIG. 3B and FIG. 5C. Illustration of L3 is omitted in FIG. 3B, to clarify the illustration.

The relation "L2>L1>L3" applies in the configuration described above.

Each of the lengths described above is a length of the elastic region in the longitudinal axis C direction in each of the states, and a distance of the elastic member from the distal end portion to the proximal end portion.

As illustrated in FIG. 3A, the elastic member 221 expands to be longer than the natural length of the elastic member 221 alone, in the maximum laid state in which the raising stand 201 is pressed by the pulling and pressing member 211 and laid to the maximum.

In the maximum laid state as illustrated in FIG. 3A, the restoring force of the elastic member 221 to restore the expanding elastic member 221 to its natural length is smaller than the pressing force of the pulling and pressing member 211 to lay down the raising stand 201. For this reason, the raising stand 201 is prevented from being unintentionally raised up by the restoring force in the maximum laid state.

In the maximum raised state as illustrated in FIG. 3B, the restoring force of the elastic member 221 to restore the contracting elastic member 221 to its natural length is smaller than the pulling force of the pulling and pressing member 211 to raise up the raising stand 201. For this reason, the raising stand 201 is prevented from being unintentionally laid down by the restoring force in the maximum raised state.

In the maximum raised state, as illustrated in FIG. 3B and FIG. 5C, when the elastic member 221 contracts, the elastic region of the elastic member 221 described above bulges outward in the radial direction of the elastic member 221 by the pulling force of the pulling and pressing member 211.

In this case, the center portion of the elastic region in the axial direction of the elastic member 221 bulges outward to the maximum, and the both end portions thereof bulge outwards to the minimum. The elastic member 221 extends in a substantially straight line shape even in the maximum raised state. In this state, the external circumferential surface of the elastic member 221 does not come to a state including many wrinkles (the elastic member does not come to a buckling state), but extends. For this reason, most of the external circumferential surface of the elastic member 221 is exposed.

The elastic member 221 is formed of, for example, a fluorine-based rubber material, a urethane-based rubber material, a silicon-based rubber material, or elastomer, in consideration of the elasticity of the elastic member 221.

In consideration of the fact that the elastic member 221 is repeatedly used and the elastic member 221 is washed, for example, the external surface of thermoplastic elastomer having more mechanical strength than that of a rubber material is coated with a fluoride resin with high chemical resistance. The thermoplastic elastomer includes, for example, a polyurethane tube. The tube including fluoride resin (coating) preferably has a thickness of, for example, 0.15 mm to 0.2 mm, in view of the space and the strength.

The internal circumferential surface of the elastic member 221 may be coated, as desired, to suppress wearing of the internal circumferential surface of the elastic member 221 due to contact with the pulling and pressing member 211.

### [Conclusion]

With the configuration described above, the coupling member 213, the attachment portion 201f, the containing portion 215 (mouthpiece portion 217 and cylindrical member 219), and the elastic member 221 function as a watertightness securing unit to cover the pulling and pressing member 211 as one unitary piece, prevent the pulling and pressing member 211 from being exposed to the outside, and secure watertightness. In other words, the transmission mechanism 205 includes a watertightness securing unit.

### [Operation]

The inserting unit 20 is inserted into a body cavity, and the insertion object is inserted from the inserting port portion 35a into the inserting unit 20. The insertion object is inserted through the channel 35b, and inserted into the body cavity from the distal end opening portion 109. When the raising operation unit 203 is operated, the pulling and pressing member 211 is pulled or pressed, and the raising stand 201 is raised up or laid down. In this manner, the rotation angle of the raising stand 201 is adjusted, and the insertion object approaches the affected part to observe or treat the affected part.

When the inserting unit 20 is inserted into the body cavity, the inserting unit 20 contacts the contaminated liquid such as a body fluid. For this reason, the contaminated liquid adheres to the raising stand 201, the elastic member 221 covering the pulling and pressing member 211, and the coupling member 213. These members are preliminarily washed with washing tools such as gauze and a washing brush after operation, and thereafter the inserting apparatus 10 is washed and sterilized by a washing device (not illustrated).

Because the elastic member 221 covers the pulling and pressing member 211, the elastic member 221 prevents adhesion of the contaminated liquid to the pulling and pressing member 211. This configuration removes the washing work of removing the contaminated liquid adhering to the pulling and pressing member 211, and improves the washing efficiency. Because the transmission mechanism has a structure different from that of a transmission mechanism of the prior art, the structure requires no O-ring, and suppresses an increase in diameter of the distal end portion of the inserting unit 20.

The pulling and pressing member 211 and the coupling member 213 are connected with each other in a watertight state. This point is also applicable to the connections between the pulling and pressing member 211 and the raising stand 201, between the mouthpiece portion 217 and the cylindrical member 219, between the mouthpiece portion 217 and the wall portion 111, between the elastic member 221 and the coupling member 213, and between the elastic member 221 and the mouthpiece portion 217. This configuration prevents the contaminated liquid from infiltrating into the inside of the elastic member 221 and the contained member space portion 20a, also removes the labor required to wash the inside of the elastic member 221 and the contained member space portion 20a, and prevents the washing liquid and the disinfectant solution from infiltrating into the inside of the elastic member 221 and the contained member space portion 20a.

### [Effects]

As described above, according to the present embodiment, the elastic member 221 prevents the contaminated liquid from adhering to the pulling and pressing member 211 during the operation. With such configuration, the present embodiment removes the washing work to remove the contaminated liquid adhering to the pulling and pressing member 211, and improves the washing efficiency. Because the present embodiment has a simple structure in which the elastic member 221 covers the pulling and pressing member 211, the structure suppresses an increase in diameter of the distal end portion of the inserting unit 20.

In the present embodiment, the pulling and pressing member 211 and the coupling member 213 are connected to each other in a watertight state. This point is also applicable to the connections between the pulling and pressing member 211 and the raising stand 201, between the mouthpiece portion 217 and the cylindrical member 219, between the mouthpiece portion 217 and the wall portion 111, between the elastic member 221 and the coupling member 213, and between the elastic member 221 and the mouthpiece portion 217.

With such configuration, the present embodiment prevents the contaminated liquid from infiltrating into the inside of the elastic member 221, the inside of the mouthpiece portion 217, the inside of the cylindrical member 219, and the contained member space portion 20a, removes the labor required to wash the inside of the elastic member 221 and the contained member space portion 20a, and prevents the washing liquid and the disinfectant solution from infiltrating into the inside of the elastic member 221 and the contained member space portion 20a. Accordingly, the present embodiment reduces the washing and sterilizing time.

In the present embodiment, the restoring force of the elastic member 221 to contract the elastic member 221 to restore the expanding elastic member 221 to its natural length is smaller than the pressing force of the pulling and pressing member 211 to lay down the raising stand 201. For this reason, the present embodiment prevents the raising stand 201 from being unintentionally raised up by the restoring force even in the maximum laid state.

With such structure, even when the inserting unit 20 is bent in various shapes, the present embodiment prevents the raising stand 201 from being unintentionally raised up, and enables insertion and extraction of the insertion object smoothly from the distal end opening portion 109 to the outside through the raising stand 201.

In the present embodiment, when the raising operation unit 203 is released from the finger in the laid state, the pulling and pressing member 211 presses the raising stand 201, and prevents unintentional raising of the raising stand 201. In other words, the raising stand 201 is pressed by the pulling and pressing member 211 to be laid down. With such configuration, for example, in a duodenal papilla excision operation, the high-frequency knife returns to a non-cutting direction by laying down the raising stand 201. The present embodiment therefore improves safety during the operation.

In the present embodiment, if the pulling and pressing member 211 breaks in the raised state, the raising stand 201 is laid down by the restoring force of the elastic member 221 to expand the elastic member 221 to restore the contracting elastic member 221 to its natural length. With such configuration, for example, in the duodenal papilla excision operation, the raising stand 201 is laid down by expansion of the elastic member 221, and the high-frequency knife returns to the non-cutting direction by laying down the raising stand 201. With such configuration, the present embodiment improves safety during the operation.

In the case where no elastic member 221 is disposed, the inserting apparatus 10 is a catheter, and the insertion object is the inserting unit 20 of an endoscope, the pulling and pressing member 211 may contact the inserting unit 20 and may wear down.

However, in the present embodiment, because the elastic member 221 covers the pulling and pressing member 211, the elastic member 221 prevents the pulling and pressing member 211 from contacting the insertion object and wearing down in the communication portion 107, and the present embodiment improves the durability of the inserting unit 20.

In the present embodiment, when the elastic member 221 is exchangeable, the elastic member 221 can be removed and discarded without washing, after the operation. The elastic member 221 may be washed in the removed state, without being discarded. With such configuration, the prevent embodiment improves the washing efficiency.

The following is explanation of modifications of the members of the present embodiment.

### [Modification of Length of Elastic Member 221]

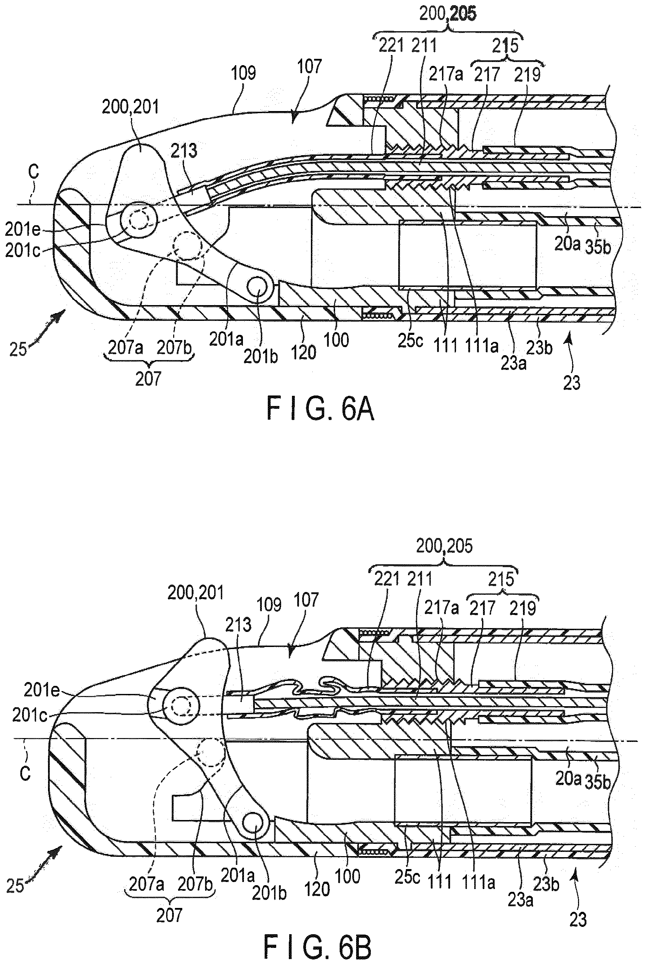

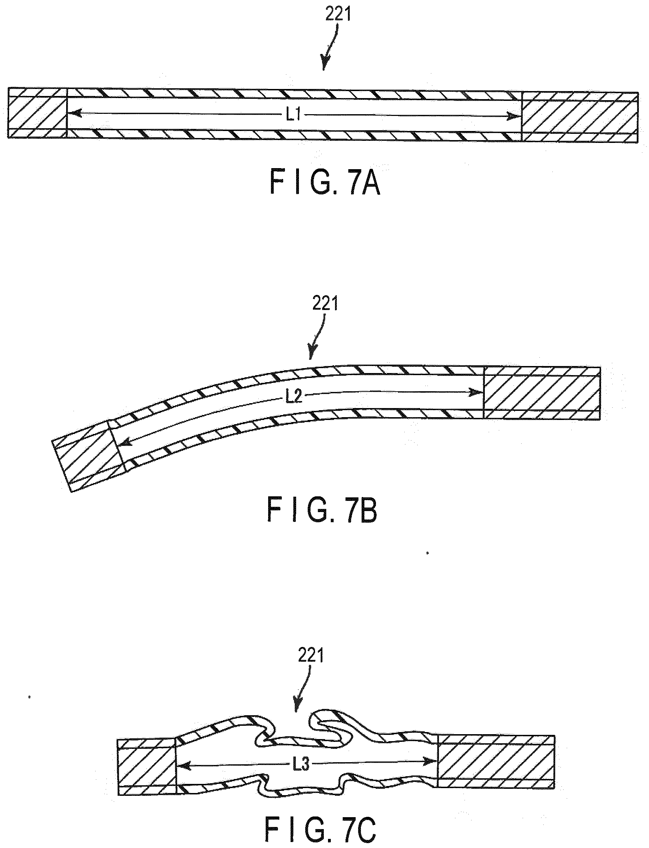

In the present modification, as illustrated in FIG. 6A, FIG. 6B, FIG. 7A, FIG. 7B, and FIG. 7C, in the laid state, the elastic member 221 contracts to be shorter than its natural length. In the raised state, the elastic member 221 contracts to be further shorter than that in the laid state, because the pulling force of the pulling and pressing member 211 acts on the elastic member 221 via the raising stand 201 and the coupling member 213.

In the present modification, the relation "L1>L2>L3" applies. To clarify the illustration, the illustration of L2 is omitted in FIG. 6A, and the illustration of L3 is omitted in FIG. 6B.

As illustrated in FIG. 6A, the elastic member 221 contracts to be shorter than the natural length of the elastic member 221 alone, even in the maximum laid state in which the raising stand 201 is pressed by the pulling and pressing member 211 and laid down to the maximum.

In the maximum laid state as illustrated in FIG. 6A, the restoring force of the elastic member 221 to restore the contracting elastic member 221 to its natural length acts on the raising stand 201 to lay down the raising stand 201, together with the pressing force of the pulling and pressing member 211 to lay down the raising stand 201. This structure prevents the raising stand 201 from being unintentionally raised up, even when the inserting unit 20 is bent in various shapes. This configuration enables insertion and extraction of the insertion object smoothly from the distal end opening portion 109 to the outside through the raising stand 201.

In the maximum raised state as illustrated in FIG. 6B, the restoring force of the elastic member 221 to restore the contracting elastic member 221 to its natural length is smaller than the pulling force of the pulling and pressing member 211 to raise up the raising stand 201. For this reason, the raising stand 201 is prevented from being unintentionally laid down.

In this case, in the raised state as illustrated in FIG. 6B, the elastic member 221 irregularly contracts in the axial direction of the elastic member 221 and is irregularly folded up, by pulling of the pulling and pressing member 211.

### [Modification of Elastic Member 221]

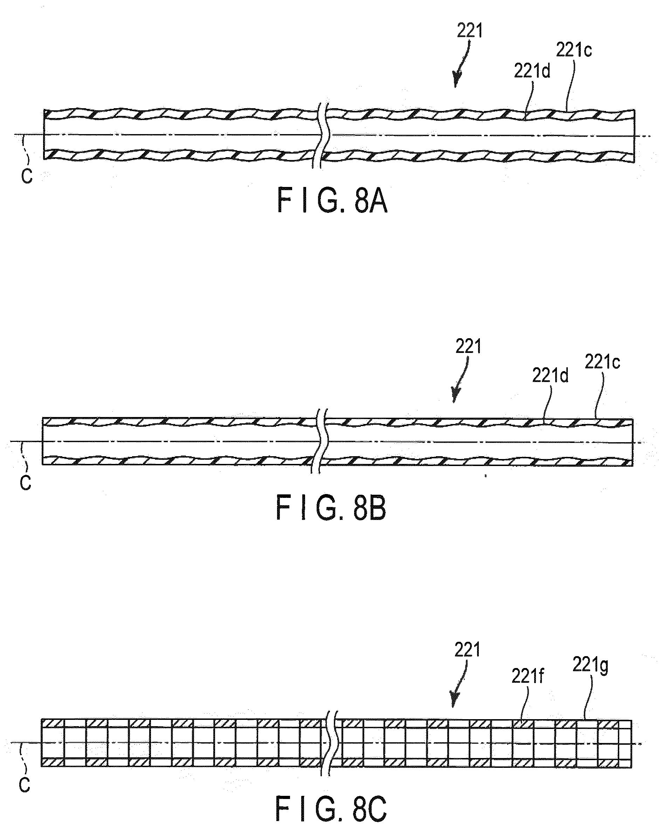

As in Modification 1 illustrated in FIG. 8A, the elastic member 221 includes a wave-shaped external circumferential surface 221c and a wave-shaped internal circumferential surface 221d, in its natural state.

As in Modification 2 illustrated in FIG. 8B, the elastic member 221 includes a substantially flat and smooth shaped external circumferential surface 221c and a wave-shaped internal circumferential surface 221d, in its natural state.

As in Modification 3 illustrated in FIG. 8C, the elastic member 221 includes hard portions 221f and soft portions 221g that are alternately arranged along the axial direction of the elastic member 221. The hard portions 221f have low elasticity, and the soft portions 221g have high elasticity. Modification 3 may be combined with Modification 1 or 2.

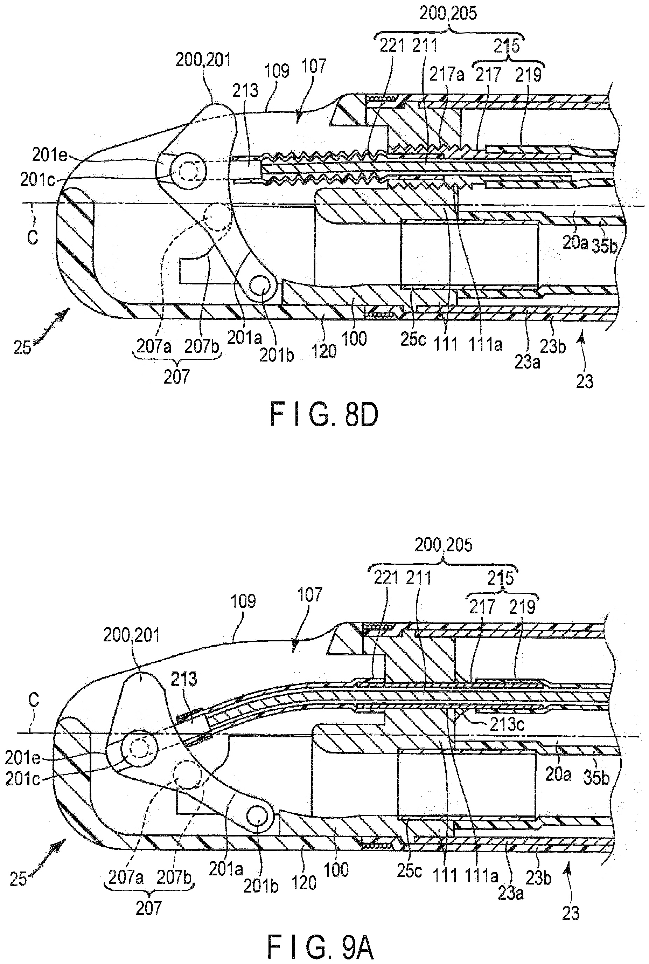

With Modifications 1, 2, and 3 described above, as illustrated in FIG. 8D, the elastic member 221 is enabled to contract substantially regularly, that is, uniformly without irregularity, in the raised state, for example. With such configuration, the present modifications improve the durability of the elastic member 221 when expansion and contraction are repeated.

In Modification 2, because the external circumferential surface 221c has a substantially flat and smooth shape, the washing liquid can be uniformly sprayed on the external circumferential surface 221c. This structure maintains the washing efficiency.

### [Modification 1 of Fixing of Elastic Member 221 in Watertight State]

As illustrated in FIG. 9A, the distal end of the mouthpiece portion 217 extends through the wall hole portion 111a of the wall portion 111, and is disposed in the communicating portion 107.

In the state where the elastic member 221 communicates with the mouthpiece portion 217 and the proximal end portion of the elastic member 221 covers the distal end of the mouthpiece portion 217, the distal end of the mouthpiece portion 217 is fixed in the watertight state to the proximal end portion of the elastic member 221. With such configuration, the internal circumferential surface of the proximal end portion is adhered and fixed to the external circumferential surface of the mouthpiece portion 217.

The proximal end portion of the elastic member 221 is adhered and fixed to the wall portion 111 on the communicating portion 107 side, and the contained member space portion 20a is kept watertight for the communicating portion 107. The mouthpiece portion 217 is not screwed into the main body portion 100 but is merely inserted. In this state, the mouthpiece portion 217 is adhered and fixed to the wall portion 111 on the contained member space portion 20a side with the adhesive 213c, and the contained member space portion 20a is kept watertight for the communicating portion 107.

The distal end portion of the elastic member 221 is connected in the watertight state to the proximal end portion of the coupling member 213. Specifically, in the state where the coupling member 213 communicates with the elastic member 221, the proximal end portion of the coupling member 213 is inserted into the distal end portion of the elastic member 221. In addition, the external circumferential surface of the proximal end portion of the coupling member 213 is adhered to the internal circumferential surface of the distal end portion of the elastic member 221. In addition, the distal end portion is tightly bound to the proximal end portion with a thread or the like.

### [Modification 2 of Fixing of Elastic Member 221 in Watertight State]

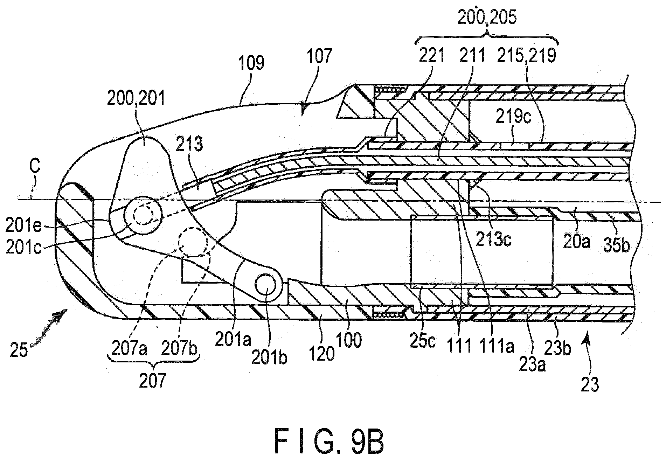

As illustrated in FIG. 9B, in the present modification, the mouthpiece portion 217 is omitted, and the containing portion 215 includes the cylindrical member 219 including a distal end portion that is fixed in a watertight state to the wall hole portion 111a of the wall portion 111, and a proximal end portion disposed in the contained member space portion 20a.

The distal end of the cylindrical member 219 extends through the wall hole portion 111a of the wall portion 111, and is disposed in the communicating portion 107. The distal end portion is adhered to the wall hole portion 111a, and has secured watertightness. The cylindrical member 219 is adhered and fixed to the wall portion 111 on the contained member space portion 20a side with the adhesive 213c, and the contained member space portion 20a has secured watertightness for the communicating portion 107. The guide hole portion is disposed inside the cylindrical member 219.

In the state where the elastic member 221 communicates with the cylindrical member 219 and the proximal end portion of the elastic member 221 covers the distal end of the cylindrical member 219, the distal end of the cylindrical member 219 is fixed in a watertight state to the proximal end portion of the elastic member 221, in the state of being inserted into the proximal end portion of the elastic member 221. With such configuration, the internal circumferential surface of the proximal end portion is adhered and fixed to the external circumferential surface of the cylindrical member 219.

The proximal end portion of the elastic member 221 is adhered and fixed to the wall portion 111 on the communicating portion 107 side, and the contained member space portion 20a has secured watertightness for the communicating portion 107.

The cylindrical member 219 includes a through hole portion 219c that is disposed in the contained member space portion 20a, disposed in the circumferential surface of the cylindrical member 219, and extends through the circumferential surface of the cylindrical member 219 in the thickness direction of the cylindrical member 219. The inside and the outside of the cylindrical member 219 communicate with each other through the through hole 219c. The through hole portion 219c is disposed on the main body portion 100 side beyond the raising operation unit 203, more specifically, in the vicinity of the elastic member 221.

Suppose that the elastic member 221 is broken and a through hole portion (not illustrated) is formed in the circumferential surface of the elastic member 221. In a water leakage check, in which the inside of the inserting unit 20 is pressurized and the inserting unit 20 is submerged in water to check for air bubbles, the configuration requires much time for pressurizing the elastic member 221 in the case where the through hole portion is distant from the main body portion 100.

However, such configuration enables prompt pressurization of the elastic member 221 through the through hole portion 219c. Such configuration also enables prompt checking of the position and the size of a through hole portion (not illustrated) formed in the elastic member 221.

The present invention is not limited to the embodiments described above, but may be carried out with the constituent elements modified within a range not departing from the gist of the invention. Various inventions can be obtained by proper combinations of constituent elements disclosed in the embodiments described above.

## Claims

1. A transmission mechanism coupled to a raising stand and a raising operation unit operating the raising stand, and transmitting an operating force of the raising operation unit to the raising stand, the raising stand raising up a tool inserted through an inserting unit inserted into a lumen from an opening portion disposed in the inserting unit by rotation, comprising:
a pulling and pressing member including a distal end portion coupled with the raising stand and a proximal end portion coupled with the raising operation unit, and pulling or pressing the raising stand by an operation of the raising operation unit to rotate the raising stand;
a containing portion containing the pulling and pressing member with the distal end portion of the pulling and pressing member projecting therefrom; and
a cylindrical elastic member including a distal end portion connected in a watertight state to the raising stand, and a proximal end portion connected in a watertight state to the containing portion, and expanding and contracting, the distal end portion side of the pulling and pressing member projecting from the containing portion is inserted into the cylindrical elastic member.

2. The transmission mechanism according to claim 1, wherein the distal end portion of the pulling and pressing member is directly coupled with the raising stand, or indirectly coupled with the raising stand via a coupling member coupled with the raising stand and the distal end portion of the pulling and pressing member,
when the distal end portion of the pulling and pressing member is directly coupled with the raising stand, the distal end portion of the elastic member is connected in a watertight state to the raising stand, and
when the distal end portion of the pulling and pressing member is coupled with the coupling member, the distal end portion of the elastic member is connected in a watertight state to the coupling member.

3. The transmission mechanism according to claim 2, wherein the elastic member expands to be longer than a natural length of the elastic member, in a maximum laid state in which the raising stand is pressed by the pulling and pressing member and laid down to a maximum.

4. The transmission mechanism according to claim 3, wherein a restoring force of the elastic member to restore the expanding elastic member to the natural length is smaller than a pressing force of the pulling and pressing member to lay down the raising stand, in the maximum laid state, and
a restoring force of the elastic member to restore the contracting elastic member to the natural length is smaller than a pulling force of the pulling and pressing member to raise up the raising stand, in a maximum raised state.

5. The transmission mechanism according to claim 2, wherein the elastic member contracts to be shorter than a natural length of the elastic member, in a maximum laid state in which the raising stand is pressed by the pulling and pressing member and laid down to a maximum.

6. The transmission mechanism according to claim 5, wherein, in the maximum laid state, a restoring force of the elastic member to restore the contracting elastic member to the natural length acts on the raising stand to lay down the raising stand, together with a pressing force of the pulling and pressing member to lay down the raising stand, and
a restoring force of the elastic member to restore the contracting elastic member to the natural length is smaller than a pulling force of the pulling and pressing member to raise up the raising stand, in a maximum raised state.

7. The transmission mechanism according to claim 2, wherein the elastic member includes a wave-shaped external circumferential surface and a wave-shaped internal circumferential surface.

8. The transmission mechanism according to claim 7, wherein the elastic member includes a substantially flat and smooth shaped external circumferential surface and a wave-shaped internal circumferential surface.

9. The transmission mechanism according to claim 2, wherein the elastic member includes hard portions and soft portions arranged alternately along an axial direction of the elastic member.

10. The transmission mechanism according to claim 2, wherein
the coupling member is bendable in a direction of being shifted from an axial direction of the pulling and pressing member, and
a distal end portion of the coupling member is inserted into an inserting port portion disposed along a side surface of the raising stand and an orthogonal direction orthogonal to the axial direction of the pulling and pressing member.

11. The transmission mechanism according to claim 10, wherein the distal end portion of the coupling member is capable of sliding the inserting port portion around an axis of the distal end portion.

12. The transmission mechanism according to claim 10, wherein the distal end portion of the coupling member is fixed in the inserting port portion.

13. The transmission mechanism according to claim 2, wherein the distal end portion of the pulling and pressing member is bent in a direction of being shifted from an axial direction of the pulling and pressing member, and directly coupled with the raising stand.

14. The transmission mechanism according to claim 2, wherein the distal end portion of the pulling and pressing member is directly coupled with the raising stand, in a state of being disposed along an axial direction of the pulling and pressing member.

15. The transmission mechanism according to claim 2, wherein
the raising table is contained in a communicating portion disposed in a distal end portion of the inserting unit and communicating with an outside, and
the containing portion is disposed in a wall portion disposed inside the inserting unit and separating the communicating portion from a space portion disposed inside the inserting unit in a watertight state from the outside.

16. The transmission mechanism according to claim 15, wherein the containing portion is further disposed in the space portion.

17. The transmission mechanism according to claim 16, wherein the containing portion includes:
a cylindrical mouthpiece portion including a distal end portion including a fixed portion fixed in a watertight state in a hole portion of the wall portion, and a proximal end portion disposed in the space portion; and
a cylindrical member is connected in a watertight state to the proximal end portion of the mouthpiece portion in a state of communicating with the mouthpiece portion.

18. The transmission mechanism according to claim 17, wherein the proximal end portion of the elastic member is inserted into the distal end portion of the mouthpiece portion, and fixed in a watertight state to the distal end portion of the mouthpiece portion.

19. The transmission mechanism according to claim 17, wherein
a distal end of the mouthpiece portion extends through the hole portion of the wall portion and is disposed in the communicating portion, and
in a state where the elastic member communicates with the mouthpiece portion and the proximal end portion of the elastic member covers the distal end of the mouthpiece portion, the distal end of the mouthpiece portion is fixed in a watertight state to the proximal end portion of the elastic member.

20. The transmission mechanism according to claim 16, wherein
the containing portion includes a cylindrical member including a distal end portion fixed in a watertight state to a wall hole portion of the wall portion, and a proximal end portion disposed in the space portion,
a distal end of the cylindrical member extends through the wall hole portion of the wall portion and is disposed in the communicating portion, and
in a state where the elastic member communicates with the cylindrical member and the proximal end portion of the elastic member covers the distal end of the cylindrical member, the distal end of the cylindrical member is fixed in a watertight state to the proximal end portion of the elastic member.

21. A raising device comprising:
a raising stand raising a tool inserting through an inserting unit inserted into a lumen from an opening portion disposed in the inserting unit by rotation;
a raising operation unit operating the raising stand; and
the transmission mechanism according to claim 1 coupled with the raising stand and the raising operation unit, and transmitting an operating force of the raising operation unit to the raising stand.

22. An inserting apparatus comprising the raising device according to claim 21.
